(19) Europäisches Patentamt
European Patent Office
Office européen des brevets

(11) Veröffentlichungsnummer : **0 385 371 B1**

(12) # EUROPÄISCHE PATENTSCHRIFT

(45) Veröffentlichungstag der Patentschrift : **01.07.92 Patentblatt 92/27**

(51) Int. Cl.$^5$ : **C07D 249/08**, A01N 43/653

(21) Anmeldenummer : **90103741.6**

(22) Anmeldetag : **26.02.90**

(54) **1-Hydroxy-1,2,4-triazolverbindungen und diese enthaltende Fungizide.**

Die Akte enthält technische Angaben, die nach dem Eingang der Anmeldung eingereicht wurden und die nicht in dieserPatentschrift enthalten sind.

(30) Priorität : **03.03.89 DE 3906771**

(43) Veröffentlichungstag der Anmeldung : **05.09.90 Patentblatt 90/36**

(45) Bekanntmachung des Hinweises auf die Patenterteilung : **01.07.92 Patentblatt 92/27**

(84) Benannte Vertragsstaaten : **AT BE CH DE DK ES FR GB GR IT LI NL SE**

(56) Entgegenhaltungen : **EP-A- 0 040 345**

(73) Patentinhaber : **BASF Aktiengesellschaft**
**Carl-Bosch-Strasse 38**
**W-6700 Ludwigshafen (DE)**

(72) Erfinder : **Baus, Ulf, Dr.**
**Keltenweg 10**
**W-6915 Dossenheim (DE)**
Erfinder : **Reuther, Wolfgang, Dr.**
**Am Pferchelhang 16**
**W-6900 Heidelberg (DE)**
Erfinder : **Lorenz, Gisela, Dr.**
**Erlenweg 13**
**W-6730 Neustadt (DE)**
Erfinder : **Ammermann, Eberhard, Dr.**
**Sachsenstrasse 3**
**W-6700 Ludwigshafen (DE)**

EP 0 385 371 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue N-Hydroxytriazolderivate, ihre Salze und Metallkomplexe, ein Verfahren zu ihrer Herstellung und ihre Verwendung als Fungizide.

Es ist bekannt, Triazolylderivate z.B. das 1-(4-Chlorphenyl)-3-(1,2,4-Triazolyl-1-methyl)-4,4-dimethyl-pentanol-3 (DE-30 18 866) als Fungizid zu verwenden. Seine Wirkung ist jedoch nicht in allen Fällen befriedigend.

Es wurde nun gefunden, daß Verbindungen der allgemeinen Formel I

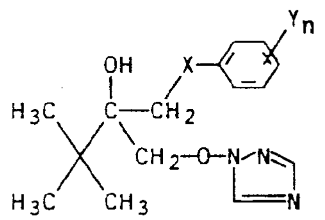

I,

in der

X $CH_2$ oder O;

Y Wasserstoff, $C_1$-$C_9$-Alkyl, $C_1$-$C_4$-Alkoxy, F, Cl, Br, Phenyl, Phenoxy, und

n 1 bis 5 bedeuten,

und ihre Salze und Komplexverbindungen, eine überraschend gute fungizide Wirkung haben.

Y bedeutet beispielsweise $C_1$-$C_9$-Alkyl, insbesondere $C_1$-$C_4$-Alkyl (Methyl, Ethyl, tert. -Butyl, Pentyl, Hexyl, Heptyl, Octyl, Nonyl), $C_1$-$C_4$-Alkoxy (Methoxy, Ethoxy, tert. -Butoxy), Halogen (Cl, Br, F),

n bedeutet beispielsweise 1, 2, 3, 4 oder 5, wobei für n größer als 1 die Bedeutungen von Y gleich oder verschieden sind, z.B. Y = 3-Chlor-4-methyl-. Salze sind beispielsweise die pflanzenverträglichen Säureadditionssalze, z.B. die Salze mit anorganischen oder organischen Säuren, wie beispielsweise die Salze der Salzsäure, Bromwasserstoffsäure, Salpetersäure, Oxalsäure, Essigsäure, Schwefelsäure, Phosphorsäure oder Dodecylbenzolsulfonsäure. Die Wirksamkeit der Salze geht auf das Kation zurück, so daß die Wahl des Anions im allgemeinen beliebig ist.

Ferner lassen sich die Verbindungen der Formel I nach bekannten Methoden in Metallkomplexe überführen. Das kann durch Umsetzung dieser Verbindungen mit Metallsalzen, z.B. Salzen der Metalle Kupfer, Zink, Eisen, Mangan oder Nickel, beispielsweise Kupfer(II)-chlorid, Zink(II)-chlorid, Eisen(III)-chlorid, Kupfer(II)-nitrat, Mangan(II)-chlorid oder Nickel(II)-bromid erfolgen.

Es wurde ferner gefunden, daß sich die Verbindungen der allgemeinen Formel I sehr leicht und in guten Ausbeuten beispielsweise durch Umsetzung von 1-Hydroxy-1,2,4-triazol mit den Verbindungen der allgemeinen Formel II,

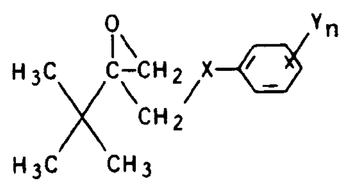

II,

in der X, Y und n die oben angegebenen Bedeutungen haben, herstellen lassen.

Die Umsetzung erfolgt z.B. in einem inerten org. Lösemittel wie Tetrahydrofuran (THF), Dimethylsulfoxid (DMSO), Dimethylformamid (DMF), Diethylether, vorzugsweise THF oder THF/Wasser-Gemisch in Gegenwart einer Base wie Triethylamin, Tributylamin, NaOH, Natriumcarbonat oder Pyridin bei Temperaturen zwischen 0 und 100°C, vorzugsweise bei Raumtemperatur (20°C). Die Verbindungen der allgemeinen Formel I entstehen als Racemate, die in üblicher Weise in ihre Isomeren getrennt werden können. Sowohl die reinen Enantiomeren als auch ihre Gemische sind Gegenstand der Erfindung und als Fungizide brauchbar.

Die Verbindungen der allgemeinen Formel II sind bekannt oder lassen sich durch bekannte Verfahren herstellen (z.B. EP 0 040 345). 1-Hydroxi-1,2,4-triazol läßt sich wie folgt herstellen:

103,5 g (1,5 mol) 1-H-1,2,4-Triazol wurden in 1344 g (12 mol) 50 %igem wäßrigem Kaliumhydroxid gelöst. Unter Eiskühlung wurden 340 g (3 mol) 30 %iges $H_2O_2$ und portionsweise 555 g (3,75 mol) Phthalsäureanhydrid zugegeben und 2 Stunden bei Raumtemperatur (20 bis 30°C) gerührt. Anschließend wurde mit ca. 35 %iger

Schwefelsäure auf einen pH-Wert unter 1,5 angesäuert, der entstandene Niederschlag abgesaugt und das Filtrat wie üblich aufgearbeitet. Man erhielt 19 g 1-Hydroxy-1,2,4-triazol Fp.: 132°C. Das ist eine Ausbeute von 15 % der Theorie.

Herstellungsbeispiel

1,7 g (20 mmol) 1-Hydroxy-1,2,4-triazol werden in 10 ml n-Butanol gelöst. Unter Rühren werden 4,81 g (20 mmol) 2-(4-Chlorphenoxi-methyl)-2-tert.-butyl-oxiran zugegeben. Die Lösung wird mehrere Stunden unter Rückflußkühlung erhitzt. Danach wird in Essigester/Ether aufgenommen, mit Wasser gewaschen und die org. Phase getrocknet. Nach Entfernen des Lösemittels erhält man 5,8 g (93 % d.Th.) eines öligen Rohproduktes (Verbindung Nr. 1).
$C_{15}H_{20}OClN_3O_2$ ber.: C 58,1 H 6,5 Cl 11,4 N 13,5
(309.61)
HNMR ($CDCl_3$): 1.09 (s, 9H); 4.17 (dd, 2H); 4.62 (dd, 2H); 6.8-7.32 (m, 4H); 7.76 (s, 1H); 8.01 (s, 1H).

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90

Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 1 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 1 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 1 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 1 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 1 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 1 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 1 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),

Ammoniak-Komplex von Zink-(N,N′-propylen-bis-dithiocarbamat),
Zink-(N,N′-propylen-bis-dithiocarbamat),
N,N′-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester
heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithiolo[4,5-b]-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N′,N′-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N′-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,

3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2′-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

Anwendungsbeispiel

Wirksamkeit gegen Weizenmehltau

Blätter von in Töpfen gewachsenen Weizenkeimlingen der Sorte "Kanzler" wurden mit wäßriger Spritzbrühe, die 80 % Wirkstoff und 20 % Emulgiermittel in der Trockensubstanz enthielt, besprüht und 24 Stunden nach dem Antrocknen des Spritzbelages mit Oidien (Sporen) des Weizenmehltaus (Erysiphe graminis var. tritici) bestäubt. Die Versuchspflanzen wurden anschließend im Gewächshaus bei Temperaturen zwischen 20 und 22°C und 75 bis 80 % relativer Luftfeuchtigkeit aufgestellt. Nach 7 Tagen wurde das Ausmaß der Mehltauentwicklung ermittelt.

Das Ergebnis zeigt, daß der Wirkstoff 1 bei der Anwendung als 0,0125 %ige (Gew.%) Spritzbrühe eine gut fungizide Wirkung zeigt (100 %).

## Patentansprüche

1. Verbindungen der allgemeinen Formel

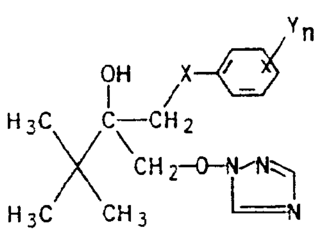

I,

in der
X $CH_2$ oder O,
Y Wasserstoff, Alkyl mit 1 bis 9 C-Atomen, $C_1$-$C_4$-Alkoxy, F, Cl, Br, Phenyl, Phenoxy, und
n 1 bis 5
bedeuten, und ihre Salze und Komplexverbindungen.

2. Verfahren zur Herstellung von Verbindungen der allgemeinen Formel I, dadurch gekennzeichnet, daß man Verbindungen der allgemeinen Formel II

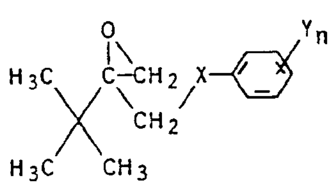

II,

in der X, Y und n die in Anspruch 1 angegebenen Bedeutungen haben, mit 1-Hydroxy-1,2,4-triazol umsetzt.

3. Fungizide Mittel, gekennzeichnet durch einen Gehalt an einem Trägerstoff und einer fungizid wirksamen Menge einer Verbindung der allgemeinen Formel I

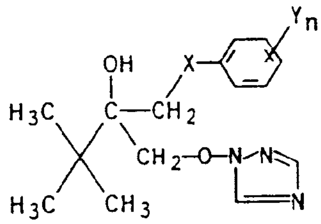

I,

in der

X $CH_2$ oder O,

Y Wasserstoff, Alkyl mit 1 bis 9 C-Atomen, $C_1$-$C_4$-Alkoxy, F, Cl, Br, Phenyl, Phenoxy, und

n 1 bis 5

bedeuten, oder ihrer Salze oder Komplexverbindungen.

4. Verfahren zur Bekämpfung von Pilzen, dadurch gekennzeichnet, daß man die Pilze oder die von Pilzbefall bedrohten Pflanzen, Saatgüter, Materialien oder Flächen behandelt mit einer fungizid wirksamen Menge einer Verbindung der allgemeinen Formel I

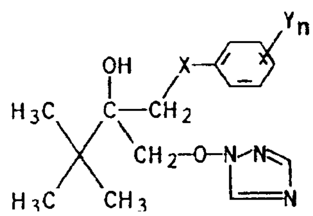

I,

in der

X $CH_2$ oder O,

Y Wasserstoff, Alkyl mit 1 bis 9 C-Atomen, $C_1$-$C_4$Alkoxy, F, Cl, Br, Phenyl, Phenoxy, und

n 1 bis 5

bedeuten, oder ihrer Salze oder Komplexverbindungen.

5. Verwendung von Verbindungen der allgemeinen Formel I gemäß Anspruch 1 als Fungizide.

6. Verbindung der allgemeinen Formel I gemäß Anspruch 1, in der X den Rest 0 und $Y_n$ 4-Chlor bedeutet.

## Claims

1. A compound of the formula

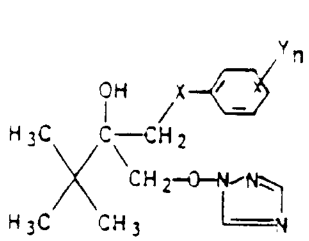

I

where

X is $CH_2$ or O,

Y is hydrogen, alkyl of 1 to 9 carbon atoms, $C_1$-$C_4$-alkoxy, F, Cl, Br, phenyl or phenoxy and

n is

from 1 to 5, and its salts and complexes.

2. A process for the preparation of a compound of the formula I, wherein a compound of the formula II

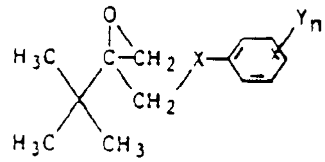

II

where X, Y and n have the meanings stated in claim 1, is reacted with 1-hydroxy-1,2,4-triazole.

3. A fungicide which contains a carrier and a fungicidal amount of a compound of the formula I

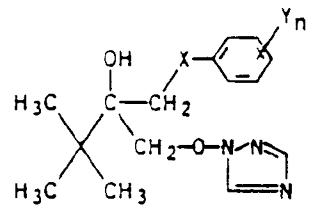

I

where
X is $CH_2$ or O,
Y is hydrogen, alkyl of 1 to 9 carbon atoms, $C_1$-$C_4$-alkoxy, F, Cl, Br, phenyl or phenoxy and
n is from 1 to 5,
or of its salts or complexes.

4. A method for controlling fungi, wherein the fungi or the plants, seeds, materials or areas threatened by fungal attack are treated with a fungicidal amount of a compound of the formula I

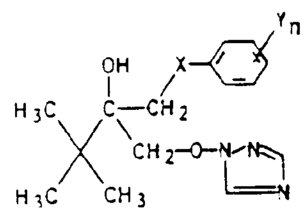

I

where
X is $CH_2$ or O,
Y is hydrogen, alkyl of 1 to 9 carbon atoms, $C_1$-$C_4$-alkoxy, F, Cl, Br, phenyl or phenoxy and
n is from 1 to 5,
or of its salts or complexes.

5. Use of a compound of the formula I as claimed in claim 1 as a fungicide.

6. A compound of the formula I as claimed in claim 1, wherein X is O and $Y_n$ is 4-chloro.

## Revendications

1. Composés de formule générale

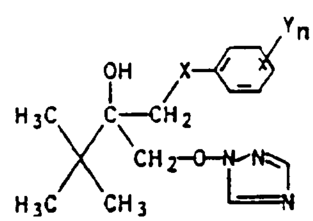

I,

dans laquelle
X représente $CH_2$ ou O,
Y représente l'hydrogène, un groupe alkyle en $C_1$-$C_9$, alcoxy en $C_1$-$C_4$, F, Cl, Br, un groupe phényle, phé-

noxy, et

n est un nombre allant de 1 à 5

et leurs sels ou complexes.

2. Procédé de préparation des composés de formule générale I, caractérisé en ce que l'on fait réagir des composés de formule générale II

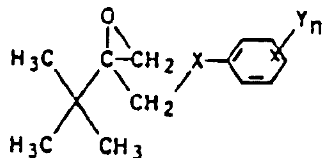

II,

dans laquelle X, Y et n ont les significations indiquées dans la revendication 1, avec le 1-hydroxy-1,2,4-triazole.

3. Produits fongicides, caractérisés en ce qu'ils contiennent un véhicule et une quantité fongicide efficace d'un composé de formule générale I

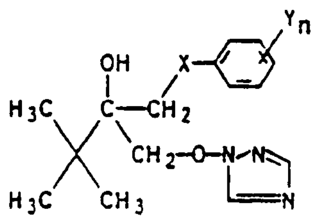

I,

dans laquelle

X représente $CH_2$ ou O,

Y représente l'hydrogène, un groupe alkyle en $C_1$-$C_9$, alcoxy en $C_1$-$C_4$, F, Cl, Br, un groupe phényle, phénoxy, et

n est un nombre allant de 1 à 5,

ou leurs sels ou complexes.

4. Procédé pour combattre les mycètes, caractérisé en ce que l'on traite les mycètes ou les végétaux, semences, matières ou surfaces menacés d'une attaque par les mycètes, par une quantité fongicide efficace d'un composé de formule générale I

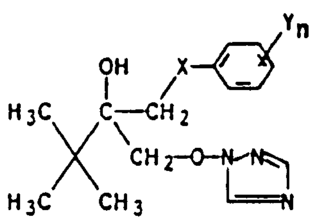

I,

dans laquelle

X représente $CH_2$ ou O,

Y représente l'hydrogène, un groupe alkyle en $C_1$-$C_9$, alcoxy en $C_1$-$C_4$, F, Cl, Br, un groupe phényle, phénoxy, et

n est un nombre allant de 1 à 5,

ou leurs sels ou complexes.

5. Utilisation des composés de formule générale I de la revendication 1 en tant que fongicides.

6. Composé de formule générale I de la revendication 1 dans laquelle X représente O et $Y_n$ représente un substituant 4-chloro.